# EUROPEAN PATENT APPLICATION

(11) **EP 3 501 462 A1**
(43) Date of publication of application: **26.06.2019**
(21) Application number: 19156072.1
(22) Date of filing: 03.10.2014
(51) Int. Cl.: A61F 7/12

(54) **FLUID CASSETTE WITH TENSIONED POLYMERIC MEMBRANES FOR PATIENT HEAT EXCHANGE SYSTEM**

(30) Priority: 14.02.2014 US 201414180613
(62) Divisional of application: 14882315.6
(71) Applicant: ZOLL Circulation, Inc., San Jose, CA 95131 (US)
(72) Inventor: Dabrowiak,, Jeremy Thomas, Redwood City, CA California 94065 (US); Pistor,, Christop Matthias, Santa Cruz, CA California 95060 (US); Pendry,, Craig Wendell, Milpitas, CA California 95035 (US); Pamichev,, Christo, Cupertino, CA California 95014 (US)
(74) Representative: Potter Clarkson

(57) **Abstract**

A working fluid cassette for an intravascular heat exchange catheter (12) includes a frame (52) holding two closely spaced, square polymeric membranes (66, 68) in tension. Working fluid from the catheter (12) is directed between the membranes (66, 68). The cassette is closely received between two refrigerant cold plates (30, 32) to exchange heat with the working fluid, which is circulated back to the catheter.

## Description

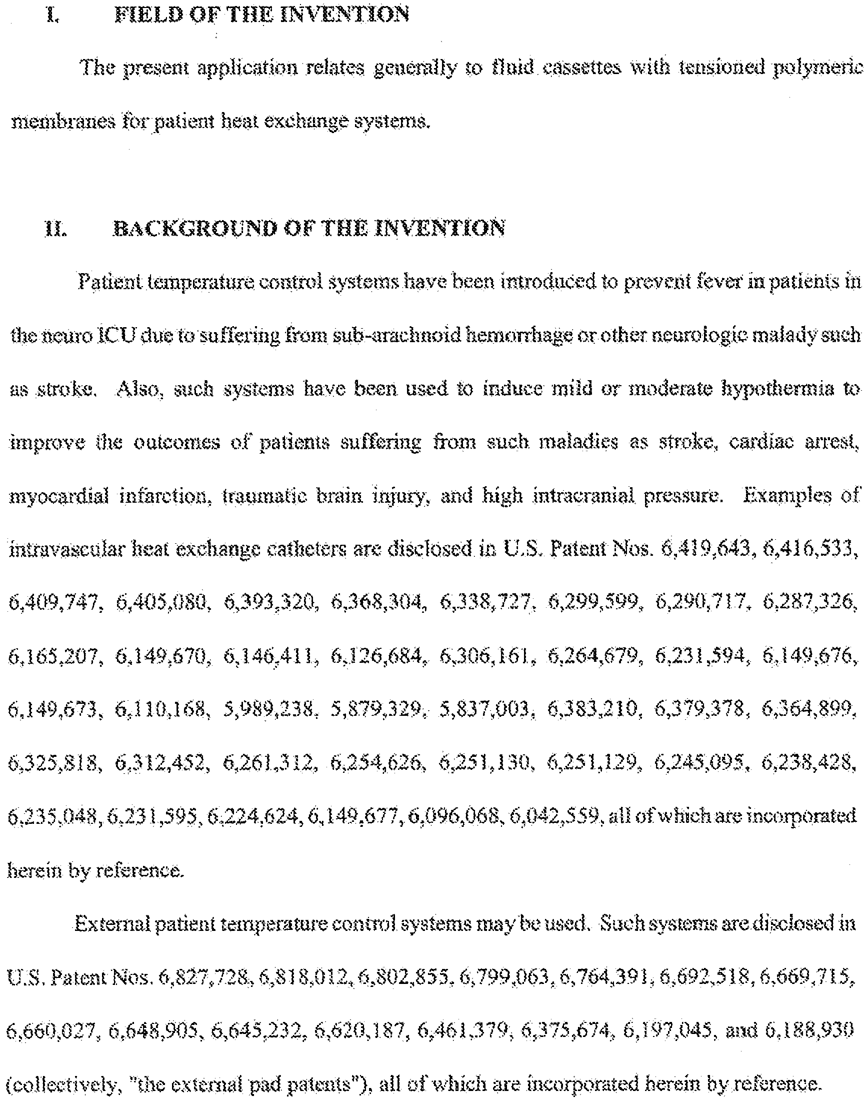

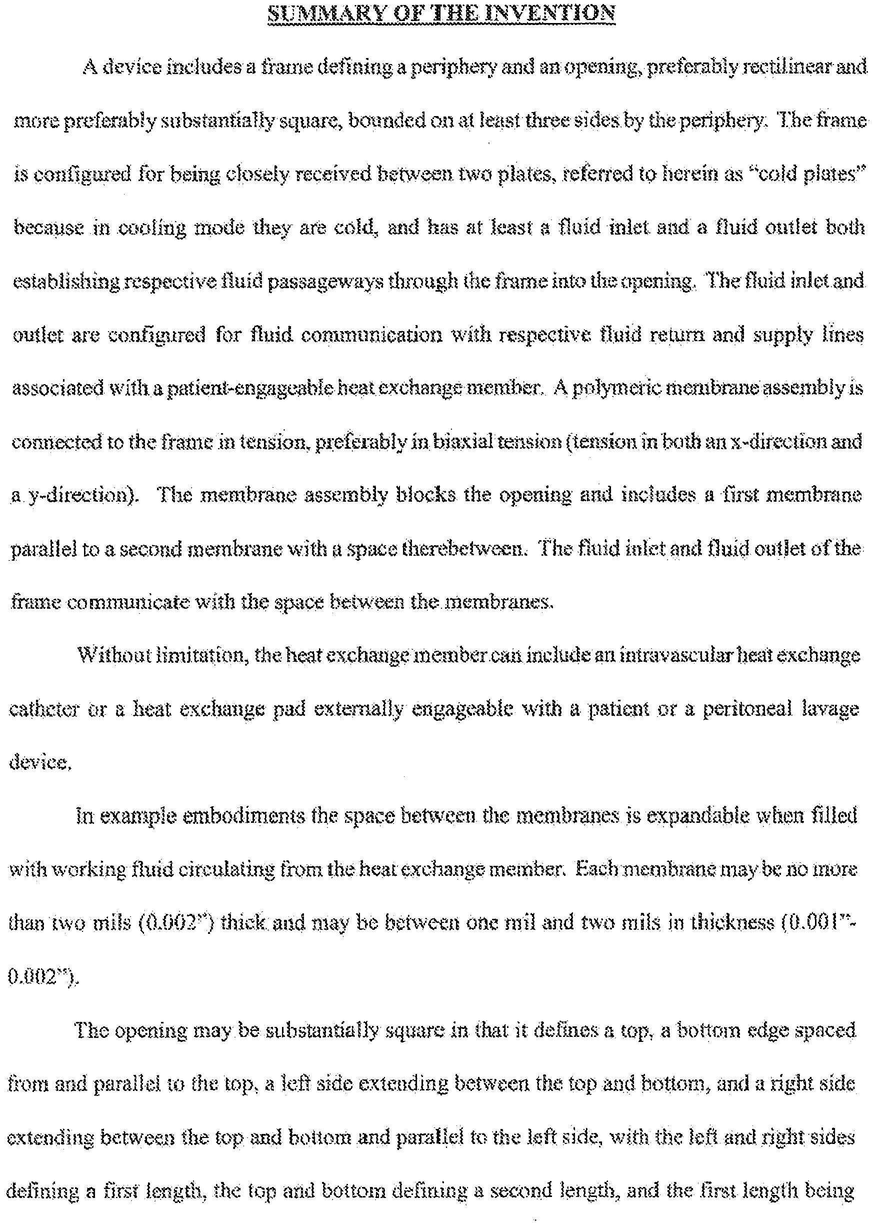

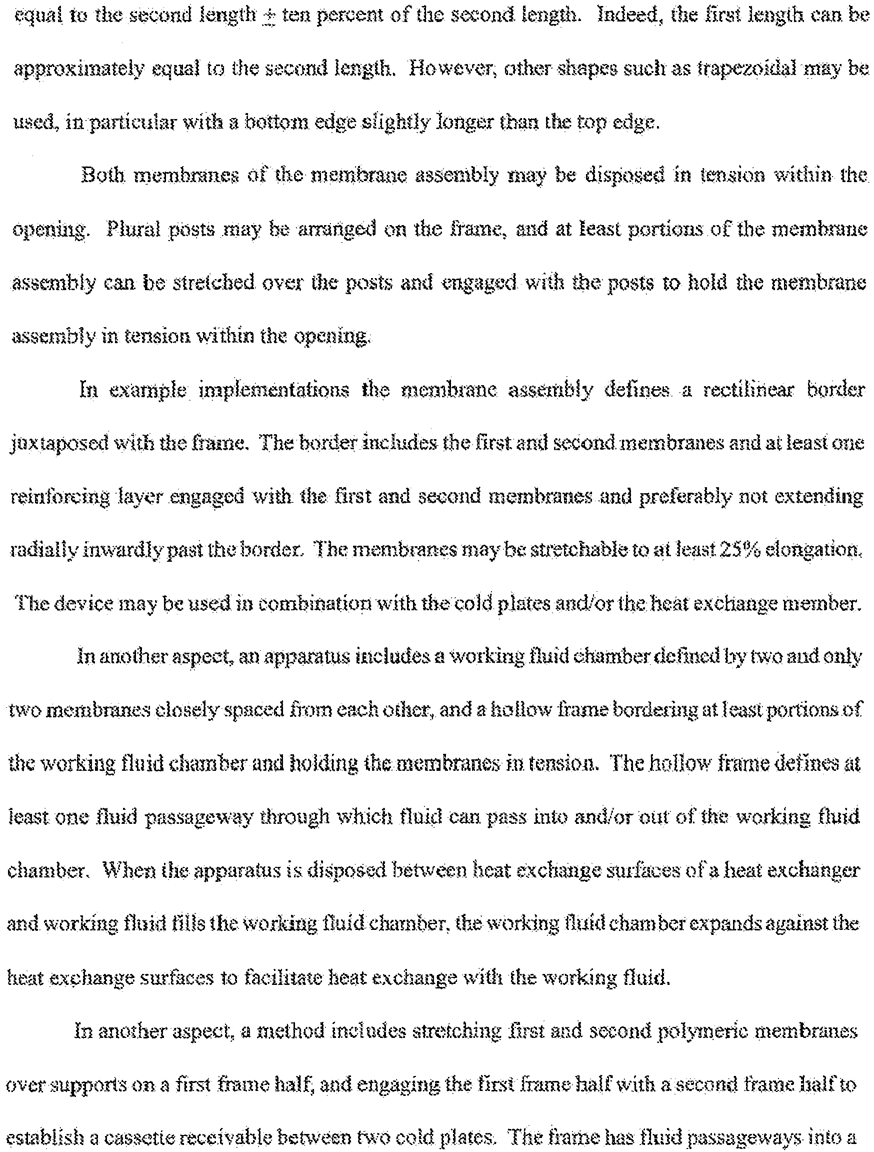

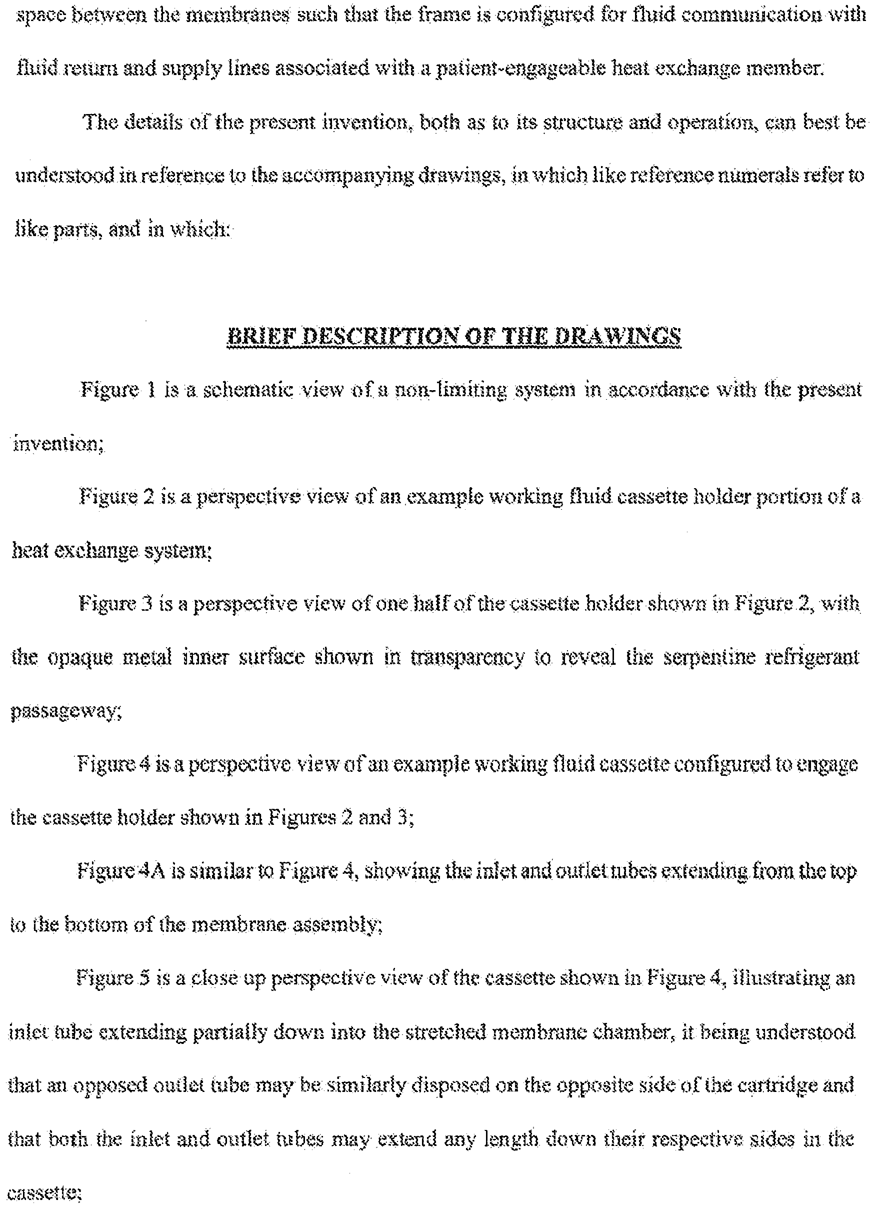

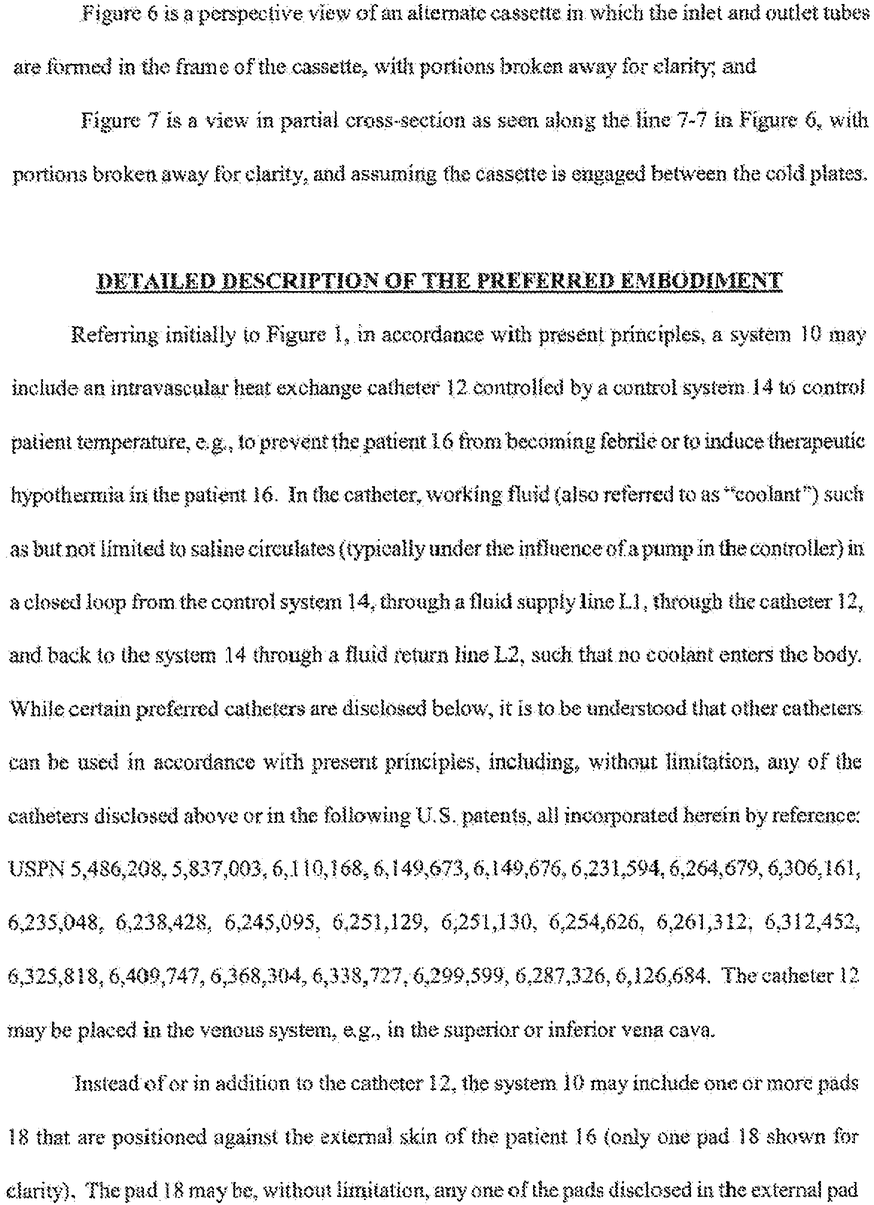

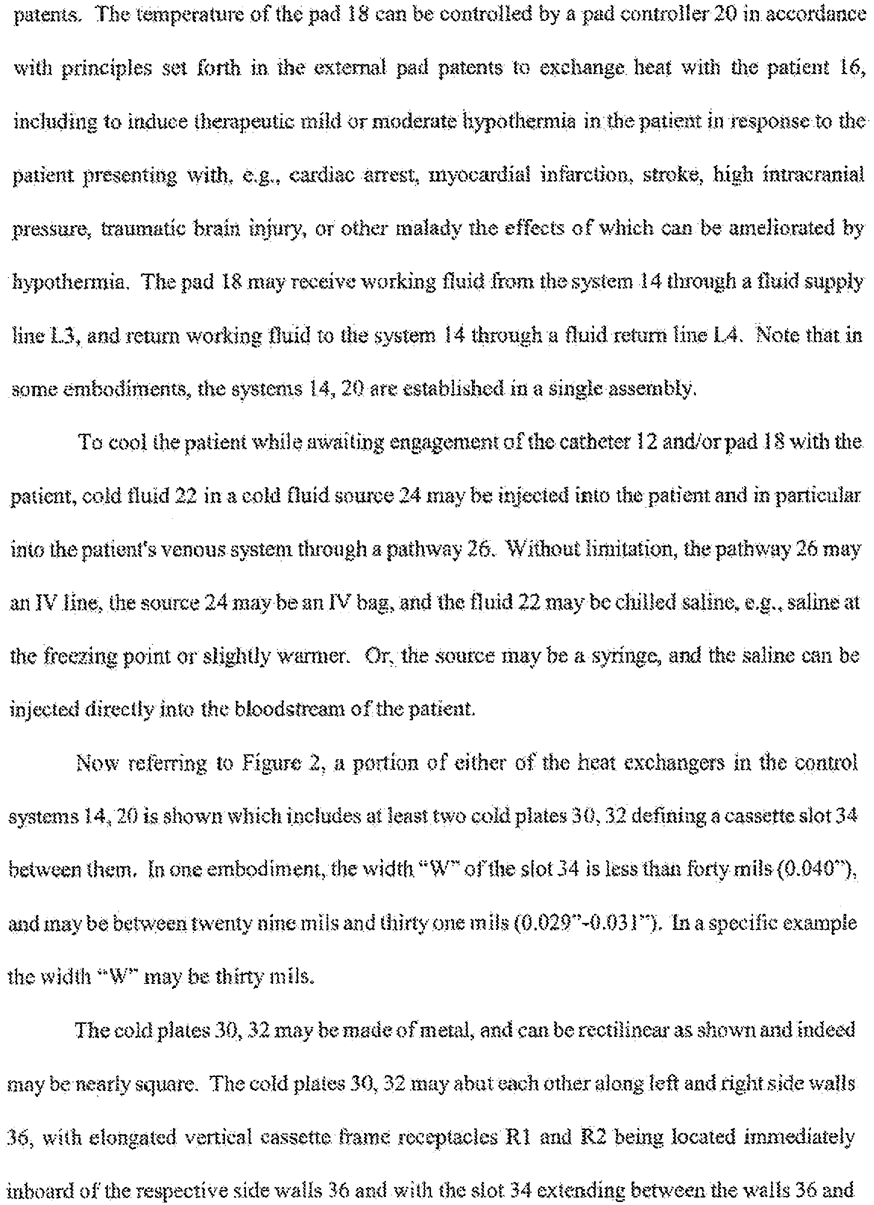

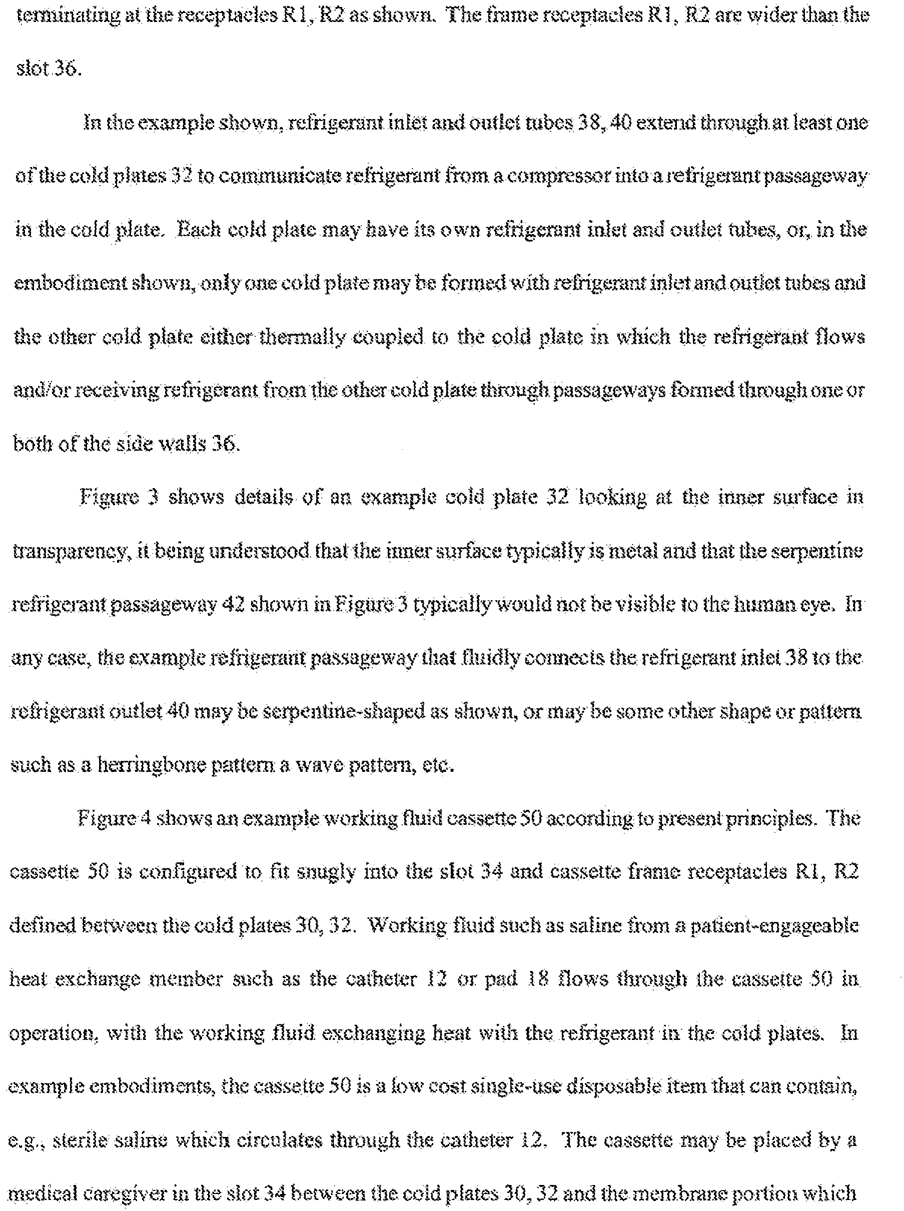

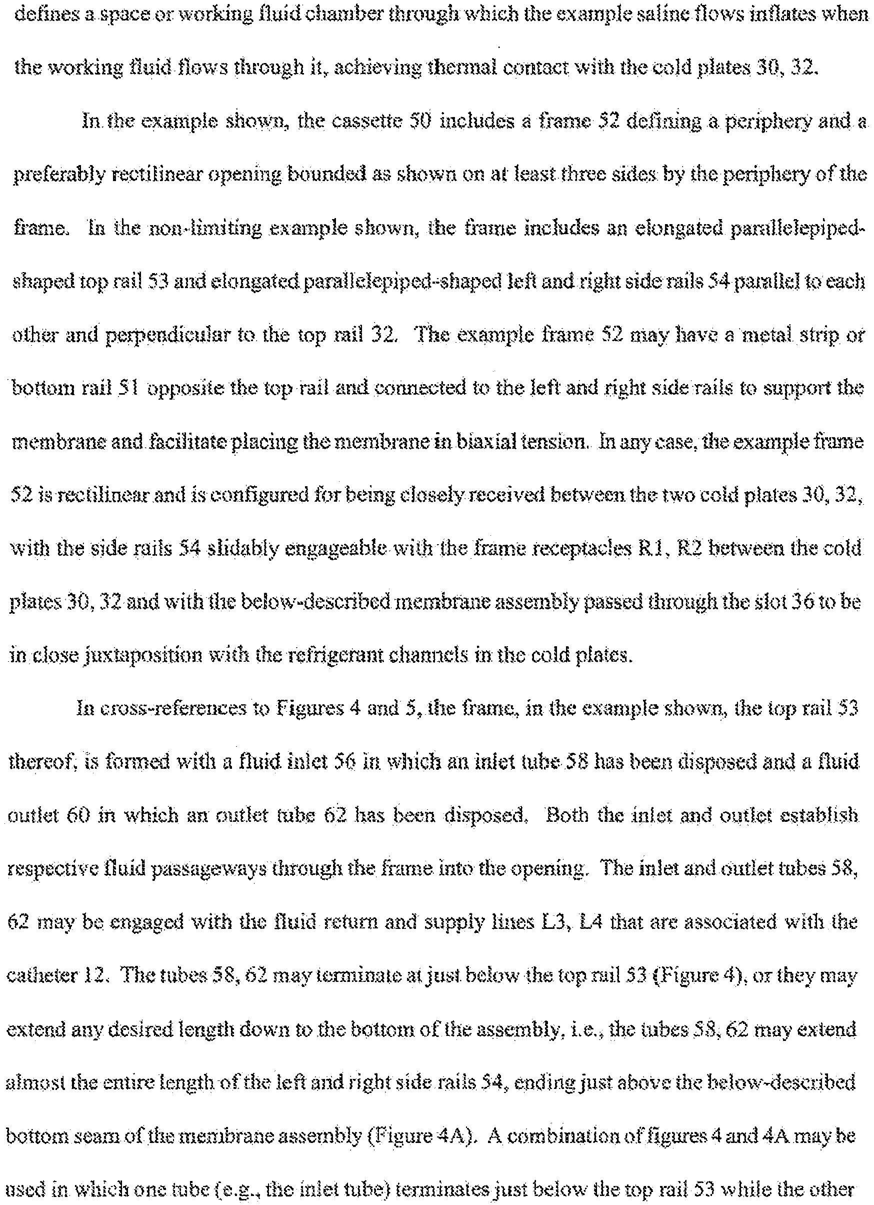

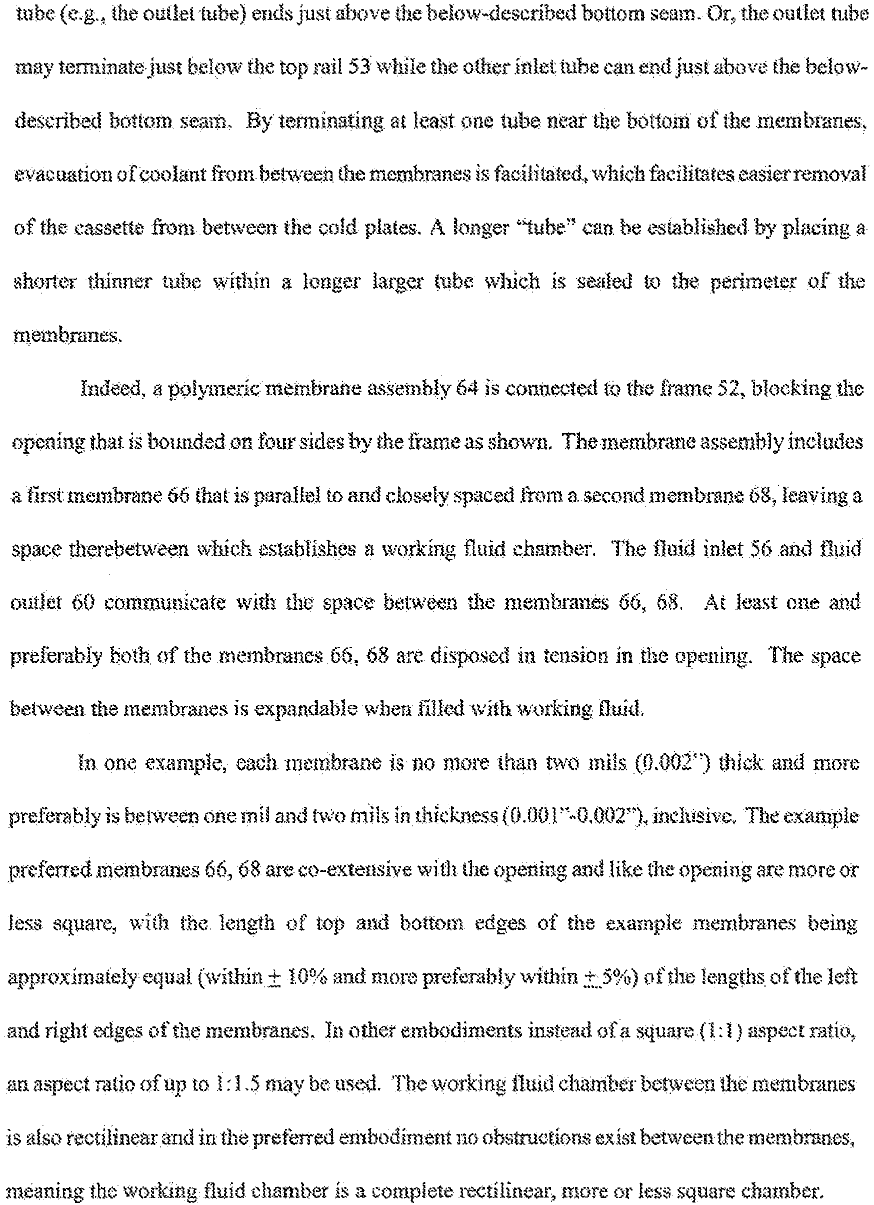

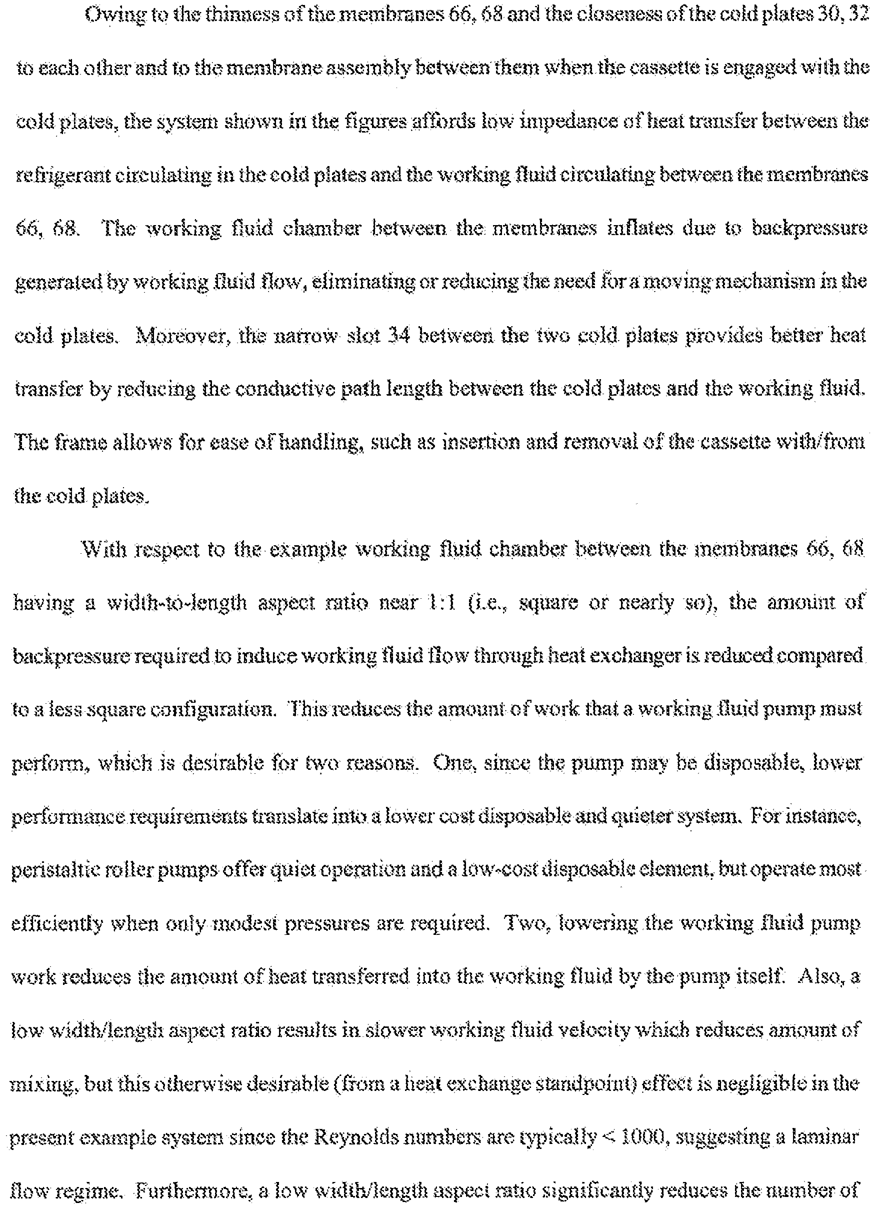

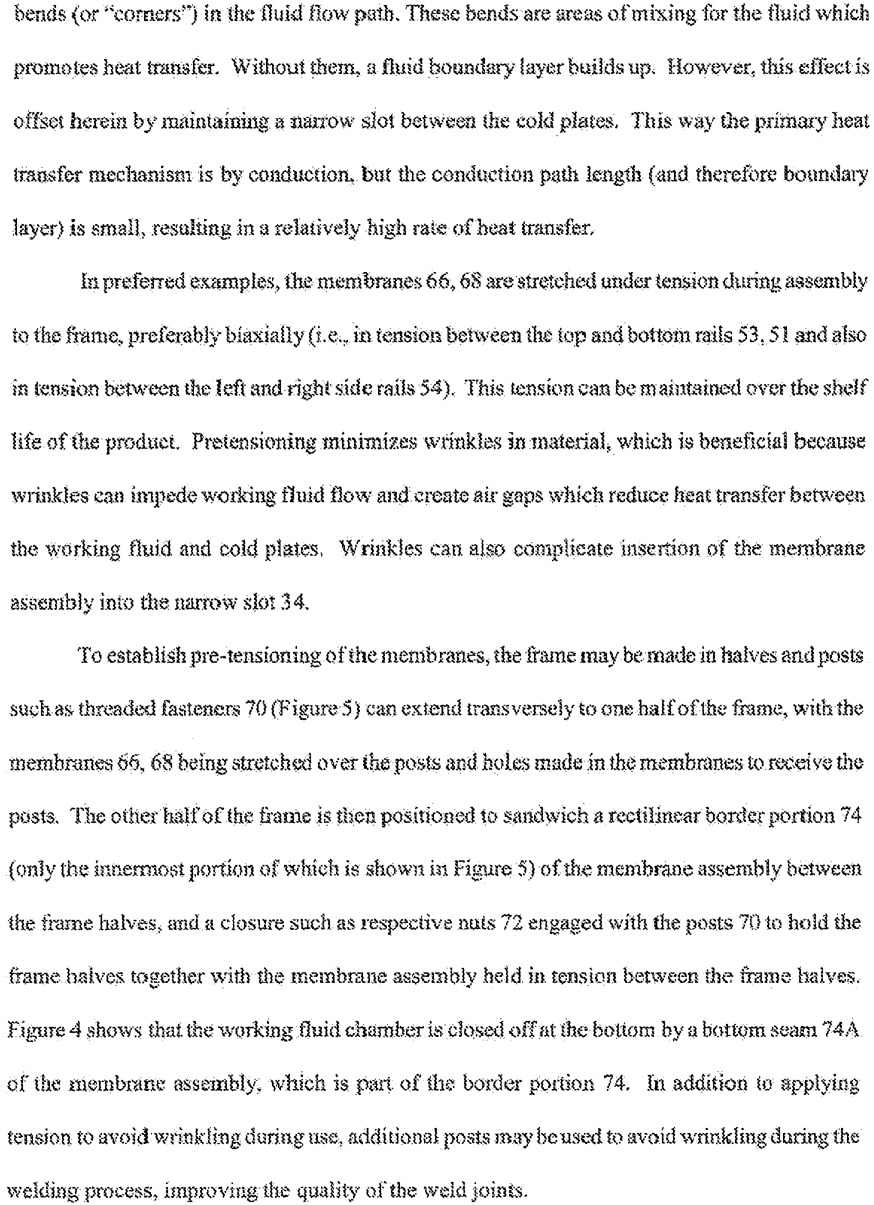

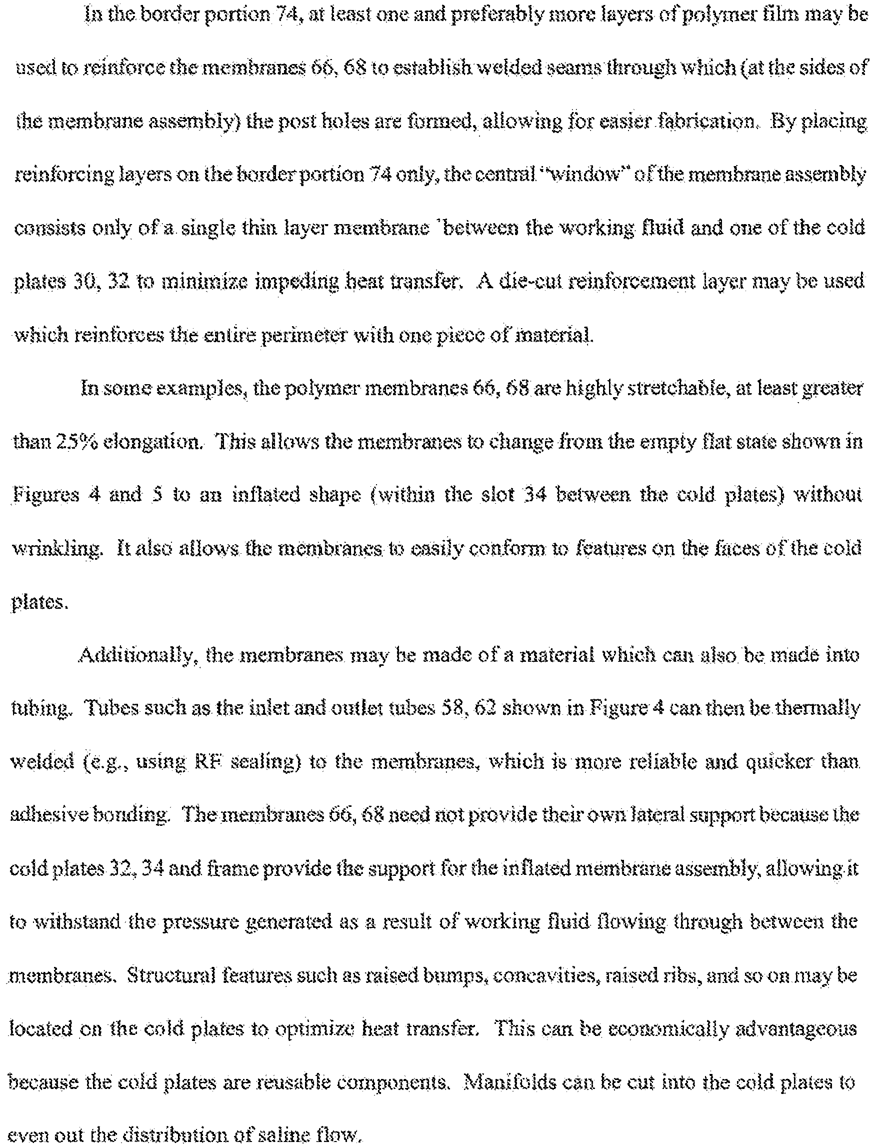

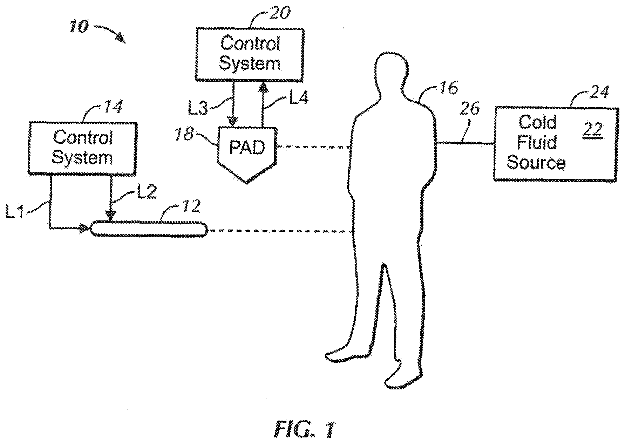

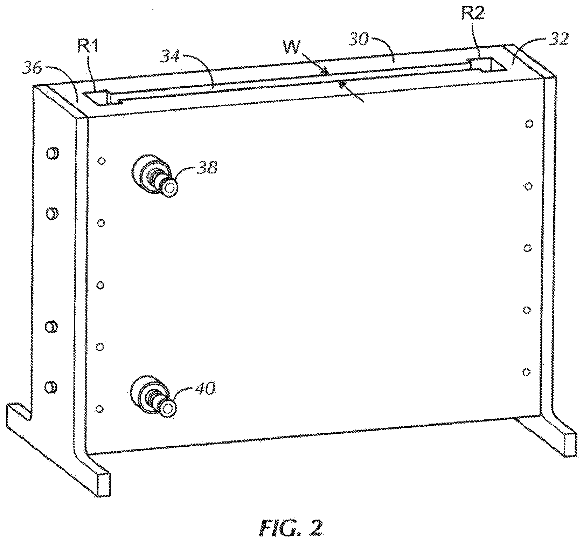

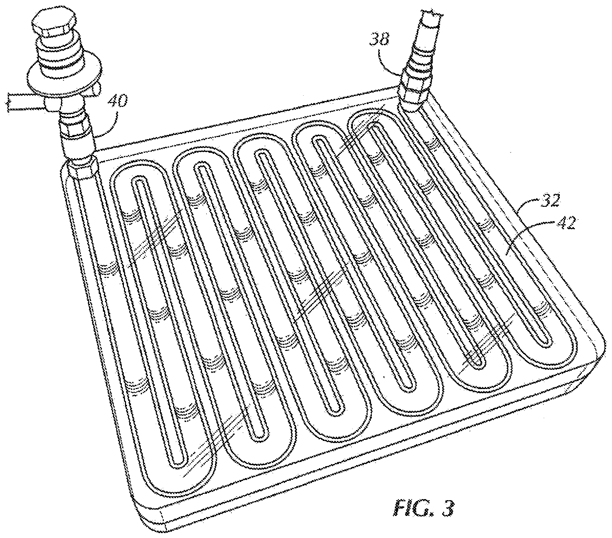

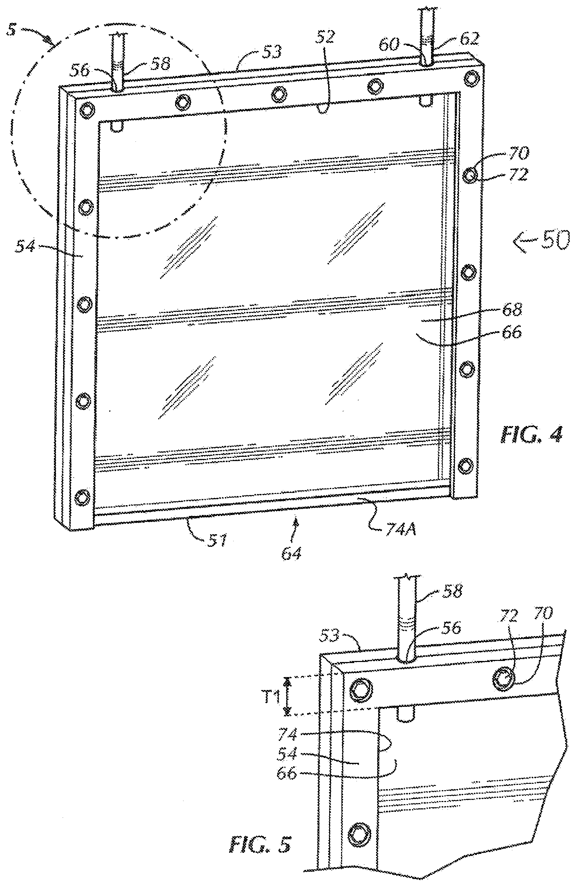

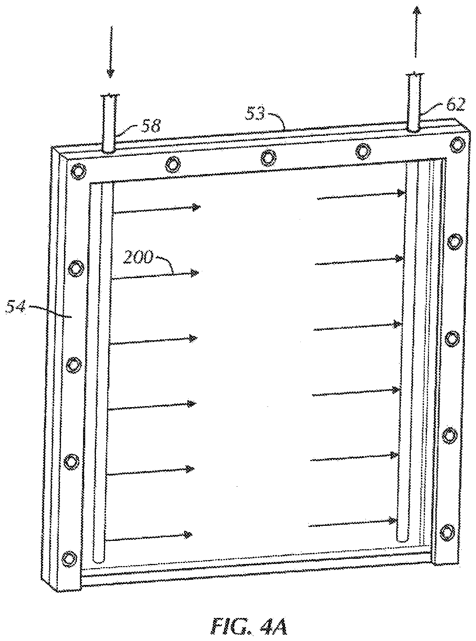

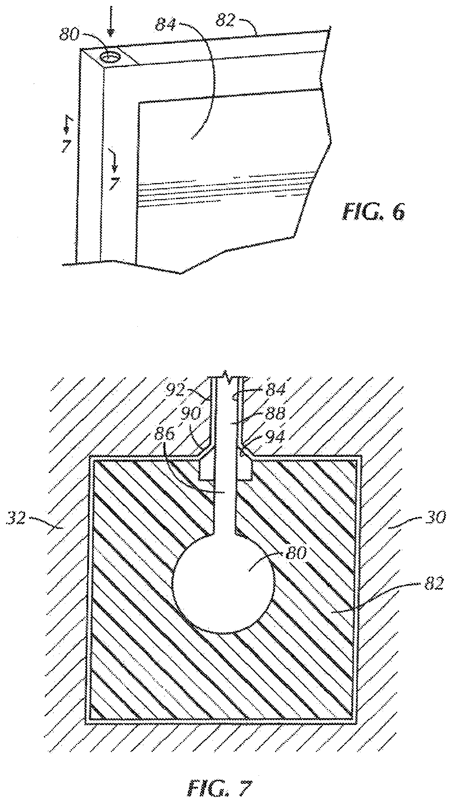

Figures 6 and 7 show that alternatively, a working fluid inlet 80 may be formed in the left rail of a frame 82 holding a membrane assembly 84 in tension. It is to be understood that a working fluid outlet may be formed in the right rail of the frame 82. The inlet 80 and outlet may extend almost the entire length of the rail if desired or may extend only part way down the rail. In any case one or more lateral channels 86 extend from the inlet 80 to the working fluid chamber 88 of the membrane assembly 84 to establish fluid communication between the inlet (and outlet) of the frame 82 and the working fluid chamber. If desired, the cold plates 30, 32 may be formed with a chamfer 90 at the start of the slot 92 in which the membrane assembly 84 is disposed, with a complementarily shaped chamfer 94 being formed in the rail of the frame 82, to accommodate any "ballooning" of the membrane assembly 84 at the frame/membrane Interface as the saline flows out of the frame into the membrane assembly.

While the particular FLUID CASSETTE WITH TENSIONED POLYMERIC MEMBRANES FOR PATIENT HEAT EXCHANGE SYSTEM is herein shown and described in detail, the scope of the present invention is to be limited by nothing other than the appended claims.

Other aspects of the invention may include any of the following.
1. Device, comprising:
   a frame (52) defining a periphery and an opening bounded on at least three sides by the periphery, the frame (52) being configured for being closely received between two cold plates (30, 32), the frame (52) having at least a fluid inlet (56) and a fluid outlet (60) both establishing respective fluid passageways through the frame (52) into the opening, the fluid inlet (56) and outlet (60) being configured for fluid communication with respective fluid return and supply lines (L3, L4) associated with a patient-engageable heat exchange member (12, 18); and
   a polymeric membrane assembly (64) connected to the frame (52) and blocking the opening, the membrane assembly (64) including a first membrane (66) parallel to a second membrane (68) with a space therebetween, the fluid inlet (56) and fluid outlet (60) communicating with the space between the membranes, at least one of the membranes (66,68) being disposed in tension in the opening.
2. The device of aspect 1, wherein the heat exchange member includes an intravascular heat exchange catheter (12).
3. The device of aspect 1, wherein the heat exchange member includes a heat exchange pad (18) externally engageable with a patient.
4. The device of aspect 1, wherein the space is expandable when filled with working fluid circulating from the heat exchange member (12, 18).
5. The device of aspect 1, wherein each membrane (66,68) is no more than two mils (0.002") thick.
6. The device of aspect 1, wherein the membranes (66,68) are disposed in biaxial tension in the frame (52).
7. The device of aspect 1, wherein the opening is rectilinear.
8. The device of aspect 7, wherein the opening defines a top, a bottom edge spaced from and parallel to the top, a left side extending between the top and bottom, and a right side extending between the top and bottom and parallel to the left side, the left and right sides defining a first length, the top and bottom defining a second length, the first length being equal to the second length ± ten percent of the second length.
9. The device of aspect 8, wherein the first length is approximately equal to the second length.
10. The device of aspect 1, wherein both membranes (66, 68) of the membrane assembly (64) are disposed in tension, within the opening.
11. The device of aspect 10, comprising plural posts (70) on the frame (52), at least portions of the membrane assembly (64) being stretched over the posts (70) and being engaged with the posts (70) to hold the membrane assembly (64) in tension within the opening.
12. The device of aspect 1, wherein the membrane assembly (64) defines a rectilinear border (74) juxtaposed with the frame (52), the border (74) including the first and second membranes and at least one reinforcing layer engaged with the first and second membranes to at least in part establish the border (74).
13. The device of aspect 1, wherein the membranes (66,68) are stretchable to at least 25% elongation.
14. The device of aspect 1, comprising the cold plates (30, 32).
15. The device of aspect 14, comprising the heat exchange member (12,18).
16. Apparatus comprising:
   working fluid chamber defined by two and only two membranes (66, 68) closely spaced from each other; and
   a hollow frame (52) bordering at least portions of the working fluid chamber and holding the membranes (66, 68) in biaxial tension, the hollow frame (52) defining at least one fluid passageway (56,60) through which fluid can pass into and/or out of the working fluid chamber,
   wherein when the apparatus is disposed between heat exchange surfaces (30, 32) of a heat exchanger and working fluid fills the working fluid chamber, the working fluid chamber expands against the heat exchange surfaces (30, 32) to facilitate heat exchange with the working fluid.
17. The apparatus of aspect 16, wherein the frame (52) is substantially square and the membranes (66, 68) are polymeric membranes (66, 68) each being no more than two mils (0.002") thick.
18. The apparatus of aspect 16, comprising plural posts (70) on the frame (52), at least portions of the membranes (66,68) being stretched over the posts (70) and being engaged with the posts (70) to hold the membranes (66,68) in tension within the opening.
19. The apparatus of aspect 16, wherein the membranes (66, 68) define a rectilinear border (74) juxtaposed with the frame (52), the border (74) including the first and second membranes (66, 68) and at least one reinforcing layer engaged with the first and second membranes (66, 68) and not extending radially inwardly past the border (74).
20. Method comprising:
   stretching first and second polymeric membranes (66,68) over supports (70) on a first frame half; and
   engaging the first frame half with a second frame half to establish a cassette (50) with a frame (52) receivable between two cold plates (30,32), the frame having fluid passageways into a space between the membranes (66, 68) such that the frame (52) is configured for fluid communication with fluid return and supply lines associated with a patient-engageable heat exchange member (12, 18).

## Claims

1. An apparatus comprising a working fluid chamber (88) defined by two membranes (66, 68) closely spaced from each other, and a hollow frame (82) bordering at least portions of the working fluid chamber (88) and holding the membranes in tension, wherein the hollow frame (82) defines at least one fluid passageway (80) through which fluid can pass into or out of the working fluid chamber (88).

2. The apparatus of claim 1 in which the hollow frame (82) defines two of said fluid passageways (80), a first one of the fluid passageways comprising a working fluid inlet and a second one of the fluid passageways comprising a working fluid outlet.

3. The apparatus of claim 2 in which the working fluid inlet is formed in a first side rail (54) of the frame and the working fluid outlet is formed in a second side rail (54) opposite to the first side rail.

4. The apparatus of claim 1 in which each at least one fluid passageway comprises a working fluid inlet or a working fluid outlet and one or more lateral channels (86) extending from the inlet (80) or the outlet to the working fluid chamber (88).

5. The apparatus of claim 3 in which the inlet (80) and the outlet both extend almost the entire lengths of the respective side rails.

6. The apparatus of claim 1 further configured to be disposed between heat exchange surfaces of a heat exchanger such that when working fluid fills the working fluid chamber, the working fluid chamber expands against the heat exchange surfaces to facilitate heat exchange with the working fluid.

7. The apparatus of claim 1 comprising only two membranes.

8. The apparatus of claim 1 in which the frame (82, 52) includes an elongated parallelepiped-shaped top rail (53) and elongated parallelepiped-shaped left and right side rails (54) parallel to each other and perpendicular to the top rail (53), a metal strip or bottom rail (51) opposite the top rail and connected to the left and right side rails to support the membrane and facilitate placing the membrane in biaxial tension.

9. The apparatus of claim 2 in which the fluid inlet and fluid outlet are configured for fluid communication with respective fluid return and supply lines (L3, L4) associated with a patient-engageable heat exchange member (12, 18).

10. The apparatus of claim 1, wherein the working fluid chamber is expandable when filled with working fluid circulating therethrough.

11. The apparatus of claim 1, wherein each membrane (66, 68) is no more than two mils (0.051 mm) thick.

12. The apparatus of claim 1, comprising plural posts (70) on the frame (52), at least portions of the membranes being stretched over the posts (70) and being engaged with the posts (70) to hold the membranes (64) in tension within the opening.

13. The device of claim 1, wherein the membranes define a rectilinear border (74) juxtaposed with the frame (52), the border (74) including the first and second membranes and at least one reinforcing layer engaged with the first and second membranes to at least in part establish the border (74).

14. The device of claim 1, wherein the membranes (66,68) are stretchable to at least 25% elongation.

15. The device of claim 1 wherein the opening is rectilinear.
